# FASCICULE DE BREVET EUROPEEN

(11) **EP 0 983 087 B1**
(45) Date de publication et mention de la délivrance du brevet: **20.10.2004**
(21) Numéro de dépôt: 98925715.9
(22) Date de dépôt: 14.05.1998
(51) Int. Cl.: A61K 39/09, A61K 39/385

(54) **COMPOSITION VACCINALE MULTIVALENTE A PORTEUR MIXTE**
MEHRWERTIGE IMPSTOFFZUSAMMENSETZUNG MIT GEMISCHTEN TRÄGERN
MULTIVALENT VACCINE COMPOSITION WITH MIXED CARRIER

(30) Priorité: 14.05.1997 FR 9706210
(43) Date de publication de la demande: 08.03.2000
(73) Titulaire: Aventis Pasteur, 69367 Lyon Cedex 07 (FR)
(72) Inventeur: LEROY, Odile, F-92360 Garches (FR)
(74) Mandataire: Ayroles, Marie-Pauline
(86) Numéro de dépôt international: PCT/FR1998/000966
(87) Numéro de publication internationale: WO 1998/051339

(56) Documents cités:
- EP-A- 0 497 525
- WO-A-88/00056
- C. CHU ET AL.: "FURTHER STUDIES ON THE IMMUNOGENICITY OF HAEMOPHILUS INFLUENZAE TYPE B AND PNEUMOCOCCAL TYPE 6A POLYSACCHARIDE-PROTEIN CONJUGATES." INFECTION AND IMMUNITY, vol. 40, no. 1, avril 1983, pages 245-256, XP002056604 WASHINGTON US
- D.P. GREENBERG ET AL.: "FACTORS INFLUENCING THE IMMUNOGENICITY OF A PNEUMOCOCCAL CONJUGATE VACCINE IN INFANTS" PEDIATRIC RESEARCH, vol. 41, 2 - 6 mai 1997, page 121A XP002078190 BASEL, CH
- G.R. SIBER: "PNEUMOCOCCAL DISEASE: PROSPECTS FOR A NEW GENERATION OF VACCINES." SCIENCE, vol. 265, 2 septembre 1994, pages 1385-1387, XP000461837 WASHINGTON, DC, US
- C.C.A.M. PEETERS ET AL.: "EFFECT OF CARRIER PRIMING ON IMMUNOGENICITY OF SACCHARIDE-PROTEIN CONJUGATE VACCINES." INFECTION AND IMMUNITY, vol. 59, no. 10, octobre 1991, pages 3504-3510, XP000371706 WASHINGTON US
- R. DAGAN ET AL.: "REDUCED RESPONSE TO MULTIPLE VACCINES SHARING COMMON PROTEIN EPITOPES THAT ARE ADMINISTERED SIMULTANEOUSLY TO INFANTS." INFECTION AND IMMUNITY, vol. 66, no. 5, mai 1998, pages 2093-2098, XP002078187 WASHINGTON US
- ANDERSON P. ET AL: 'Non-interference between two protein carriers when used with the same polysaccharide for pneumococcal conjugate vacines in 2-year-old children' VACCINE vol. 21, 2003, pages 1554 - 1559
- AHMAN H. ET AL: 'Dose dependency of antibody response in infants and children to pneumococcal polysaccharides conjugated to tetanus toxoid' VACCINE vol. 17, 1999, pages 2726 - 2732
- WUORIMAA T. ET AL: 'Tolerability and immunogenicity of an 11-valent pneumococcal conjugate vaccine in adults' VACCINE vol. 19, 2001, pages 1863 - 1869
- FATTOM A. ET AL: 'Epitopic overload at the site of injection may result in suppression of the immune response to combined capsular conjugate vaccines' VACCINE vol. 17, 1999, pages 126 - 133

## Description

La présente invention a pour objet une composition pharmaceutique destinée au traitement ou à la prévention d'un certain nombre d'infections dues à des agents pathogènes tels que les bactéries, qui comprend à titre d'agent immunogène, des polyosides dérivés d'un ou de plusieurs agents pathogènes.

Les bactéries ainsi que les champignons tels que des levures incorporent des polyosides dans leur structure de surface. Ainsi la grande majorité des bactéries sont recouvertes d'un exsudat de nature polyosidique qui est fixé à la bactérie de manière plus ou moins forte mais qui n'est pas à proprement parler une enveloppe. Cet exsudat porte le nom de glycocalyx ou de capsule. D'autre part la membrane externe des bactéries gram-négatives est entre autre constituée de lipopolysaccharide (LPS). Enfin, des polyosides se retrouvent aussi dans la paroi des champignons. Ces polyosides sont en fait des antigènes de surface qui induisent une réponse immune chez un mammifère infecté.

De tels polyosides sont constitués sur la base d'unités dans lesquelles les constituants et les liaisons sont définis et qui sont caractéristiques de l'espèce bactérienne ou fongique considérée. Ces unités répétitives contiennent les épitopes, c'est-à-dire les structures déterminantes de l'antigénicité.

Les polyosides de microorganismes pathogènes sont réputés être de bons agents vaccinaux. Tels quels ils sont efficaces chez les adultes et les enfants de plus de deux ans. Par contre chez les nourrissons, certains sont peu ou pas immunogéniques et n'induisent pas de réponse immunitaire. Il est possible de surmonter ce problème, en couplant par liaison covalente les polyosides à une protéine dite porteuse telle que l'anatoxine diphthérique ou tétanique, de manière à obtenir un conjugué polyoside - protéine porteuse.

Une même composition vaccinale peut contenir plusieurs conjugués. En effet, la tendance veut que l'on rassemble plusieurs agents vaccinaux destinés à prévenir ou à traiter des infections induites par des agents pathogènes d'espèces différentes ; ceci entre autre, afin de limiter le nombre d'administrations dans la vie d'un individu. De plus, au sein d'une même espèce, il peut exister plusieurs sérogroupes / sérotypes largement représentés au plan régional ou mondial, On rappelle qu'un sérogroupe / sérotype est caractérisé entre autres par la nature du polyoside de capsule et que des polyosides de sérogroupes différents ne présentent généralement pas de réaction immunologique croisée. Dans ce cas, il peut donc être nécessaire de réunir les polyosides provenant de différents sérogroupes, afin de lutter avec efficacité contre une infection due à une seule et même espèce.

Ainsi, cela est par exemple le cas lorsque l'on cherche à vacciner à l'encontre des infections à *Streptococcus pneumoniae*. Les infections à pneumocoques sont un réel problème de santé publique notamment dans la mesure où on les rencontre dans les formes sévères de pneumonies, de septicémies et de méningites. Dans les pays industrialisés, elles affectent chaque année 30 à 100 per 100,000 enfants de moins de trois ans. Le taux de mortalité dans les cas de bactériémies et de méningites est de 15 à 30 % tandis que 5 % des enfants meurent de pneumonies.

Une étude effectuée en Finlande de 1985 à 1989 montre que 90 % des infections invasives sont dues à 8 groupes de sérogroupes / sérotypes. Les sérogroupes / sérotypes 14, 6 et 19 sont responsables de 54 % des cas, le sérotype 14 étant prédominant chez les enfants de moins de deux ans. D'autres pneumocoques fréquemment isolés appartiennent aux sérogroupes 7, 18 et 23 ; d'autres encore, plus rares appartiennent aux sérogroupes / sérotypes 9 et 4. Une distribution similaire a été mise en évidence dans d'autres pays industrialisés, en particulier aux Etats-Unis.

D'autre part, *Streptococcus pneumoniae* est responsable d'un certain nombre d'otites, infections plus bénignes mais très courantes. On évalue à environ 75 % le nombre d'enfants ayant été atteints d'une otite avant l'âge de six ans et à 30 à 50 % le nombre d'otites dues au pneumocoque. Dans les pays développés, les otites à pneumocoque sont dues au sérogroupe 19 dans 25 % des cas ; suivis par les sérogroupes / sérotypes 23 (13 %), 6 et 14 (12 %), 9 et 18 (4 %) et 4 et 1 (2 %).

Un vaccin pneumocoque contenant les polyosides de 23 sérotypes est déjà commercialisé. Ce vaccin permet de combattre de manière efficace les infections invasives chez les adultes et possède une action transitoire chez les enfants de plus de sept mois.

Les polyosides capsulaires des pneumocoques sont des antigènes T-indépendants *i*.*e*., ils peuvent induire des anticorps, de préférence du type IgM, sans l'aide des cellules T et ne sont pas capables de promouvoir une réponse rappel de type IgG. Lorsqu'ils sont couplés à une protéine porteuse, ces polyosides se révèlent alors capable d'induire une réponse T-dépendante, tout spécialement chez les nouveau-nés et devraient conférer une protection à long terme.

Des études cliniques ont été effectuées en Finlande et en Israël, avec des vaccins pneumocoque à quatre valences contenant des conjugués 6B, 9V, 18C et 23F dans lesquels le polyoside était couplé soit à de la Dt ou à de la Tt. Les doses étaient de 1, 3 ou 10 µg de polyoside par valence. Chacune de ces formulations a été administrée simultanément avec un vaccin anti *- Haemophilus* (polyribitolphosphate couplé à de la Tt ; Act-HIB commercialisé par Pasteur Mérieux Connaught) et un vaccin anti - diphtérie, tétanos, coqueluche (pour la Finlande, D.T.Coq commercialisé par KTL). De plus, ces trois administrations ont été mises en oeuvre accompagnées ou non par une administration simultanée d'un vaccin polio oral ou injectable. Elles ont été répétées deux fois à quelques semaines d'intervalle, puis une fois, un an après la primo-immunisation.

Les résultats de ces études tels que rapportés dans le tableau ci-après ont permis de mettre en évidence un effet d'interférence négative de la charge en anatoxines diphthérique et tétanique sur l'induction des anticorps anti-HiB, après la dernière immunisation.

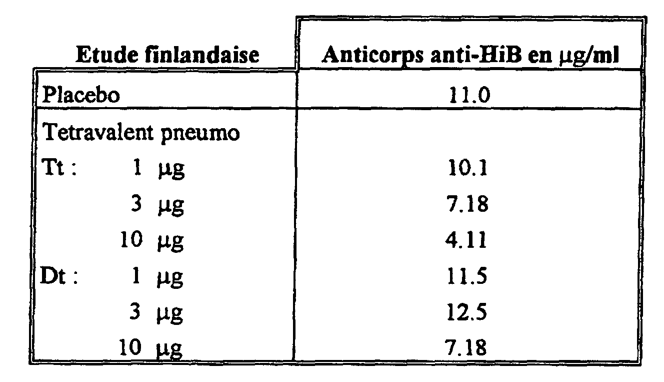

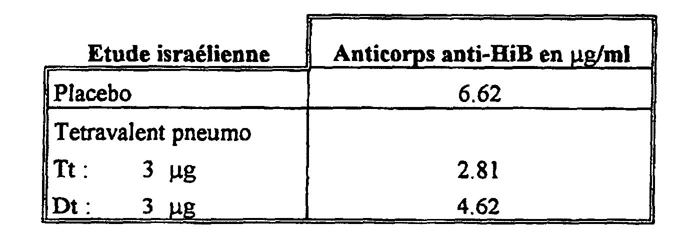

Un effet d'interférence similaire a été observé au cours d'une étude clinique en Islande dans laquelle les nourrissons avaient reçus du Pro HIBit (PRP couplé à de la Dt ; Connaught) à la place de l'Act-HIB.

D'une manière plus générale, on prévoit que, quel que soit le vaccin à base de polyosides conjugués, il existe dans le vaccin conjugué ou dans l'association ou la combinaison vaccinale administrée, une charge maximale en Dt et en Tt ou en toute autre protéine au-delà de laquelle la réponse immune à l'encontre des polyosides conjugués à cette protéine peut être diminuée. Afin de surmonter le problème que constitue le phénomène d'interférence négative dans les vaccins multivalents composés de conjugués polyosidiques, la présente demande propose d'utiliser non pas une mais au moins deux protéines porteuses afin que la charge maximale en chacune des protéines porteuses ne soit pas atteinte.

L'utilisation de deux protéines porteuse différentes dans une combinaison bivalente de conjugués polysaccharidiques a été incidemment décrite par Chu et al, Infect. Immun. (1983) 40 : 245. L'une de ces protéines porteuse (l'hémocyanine de la limule) est qualifiée de protéine porteuse non-sens ; c'est-à-dire sans utilité vaccinale. De surcroît, Chu et al ne met pas en évidence de phénomène d'interférence négative.

C'est pourquoi l'invention a pour objet une composition comprenant "n" conjugués C1 à Cn ; chaque conjugué étant composé (i) d'un polyoside, en paticulier d'un polyoside issu d'un sérotype/ sérogroupe de *Streptococcus pneumoniae*, S1 à Sn respectivement et, (ii) d'une protéine porteuse P1 à Pn respectivement ; "n" étant un nombre égal ou supérieur à 2 ; composition dans laquelle les polyosides S1 à Sn sont identiques ou différents et dans laquelle les protéines porteuses P1 à Pn sont sélectionnées de manière indépendante parmi un groupe constitué de "m" protéines porteuses A1 à Am ; "m" étant un nombre égal ou supérieur à 2 ; à condition qu'au moins une des protéines porteuses P1 à Pn soit différente des autres.

Sous un autre aspect, l'invention a aussi pour objet une composition qui comprend "n" conjugués C1 à Cn, chaque conjugué étant composé (i) d'un polyoside S1 à Sn respectivement et, (ii) d'une protéine porteuse P1 à Pn respectivement, "n" étant un nombre égal ou supérieur à 2 ; composition dans laquelle les polyosides S1 à Sn sont identiques ou différents et dans laquelle les protéines porteuses P1 à Pn sont sélectionnées de manière indépendante parmi un groupe constitué des anatoxines diphthérique (Dt) et tétanique (Tt), à condition qu'au moins une des protéines porteuses P1 à Pn soit différente des autres ; et qui est caractérisée en ce que la quantité en Dt et Tt est respectivement inférieure ou égale à 200 et 50 µg/ dose. En d'autres termes, une composition selon l'invention, comprend un ou plusieurs conjugués polyosidiques dans le ou lesquels le polyoside est couplé à l'anatoxine diphthérique (Dt) et un ou plusieurs conjugués polyosidiques dans le ou lesquels le polyoside est couplé à l'anatoxine tétanique (Tt) et est caractérisée en ce que la quantité de Dt et Tt est respectivement inférieure ou égale à 200 et 50 µg / dose.

A titre illustratif, on envisage les compositions suivantes :
(i) Une composition contenant au moins trois conjugués C1, C2, C3, ... Cn ; de formules S1-P1, S2-P2, S3-P3, ..... Sn-Pn, avec : S1 à Sn identiques entre eux et P1 à Pn toutes différentes entre elles ;
(ii) Une composition contenant au moins trois conjugués C1, C2, C3, ... Cn ; de formules S1-P1, S2-P2, S3-P3, ..... Sn-Pn, avec : S1 à Sn tous différents entre eux, P1 et P2 identiques entre elles, P3 à Pn identiques entre elles et P1 et P2 différentes de P3 à Pn ; et
(iii) Une composition contenant au moins trois conjugués C1, C2, C3, ... Cn ; de formules S1-P1, S2-P2, S3-P3, ..... Sn-Pn, avec : S1 et S2 identiques entre eux, S3 à Sn identiques entre eux, S1 et S2 différents de S3 à Sn, P1 et P3 identiques entre elles, P2 à Pn, à l'exclusion de P3, identiques entre elles et P1 et P3 différentes de P2 à Pn (-P3).

Ainsi, aux fins de la présente invention, les conjugués C1 à Cn nécessairement tous différents entre eux, peuvent l'être deux à deux soit par leur polyoside, soit par leur protéine porteuse ou par leur polyoside et leur protéine porteuse. Selon un mode particulier, les polyosides mis en oeuvre sont tous différents entre eux.

Le nombre "n" de conjugués présents dans une composition selon l'invention est égal ou supérieur à 2, et peut en particulier être égal ou supérieur à 3, 4, 6, 8, 10, 11, 12, 15 ou 20. D'une manière générale ce nombre "n" peut être déterminé par l'homme de l'art en fonction d'un certains nombre de critères notamment liés à la nature même de la composition, aux objectifs que cette composition doit permettre d'atteindre et à la population à laquelle cette composition est destinée. Par exemple, dans le cas d'une composition destinée à traiter ou à prévenir des infections à pneumocoque chez le nourrisson, on considère qu'une telle composition, pour offrir une protection de bon niveau et à l'échelle mondiale, doit contenir au moins 8, de préférence au moins 10, de manière tout particulièrement préférée au moins 11 valences, pouvant être représentées par au moins 11 conjugués ou plus.

Par "polyoside" on entend un polymère constitué d'une pluralité d'unités répétitives osidiques, notamment de plus de quatre unités répétitives, quelle que soit la longueur de la chaîne osidique et quel que soit le poids moléculaire moyen du polyoside. Ce terme englobe en particulier celui d'oligoside.

Par "conjugué" on entend un composé dans lequel un polyoside est lié de manière covalente à une protéine porteuse.

Ainsi que précédemment énoncé, une composition selon l'invention doit mettre en oeuvre au moins deux protéines porteuses. Ces protéines porteuses peuvent être choisies parmi toutes celles couramment en usage dans le domaine des vaccins. Il peut notamment s'agir de l'anatoxine diphthérique (Dt), de l'anatoxine tétanique (Tt), de la forme mutante non-toxique CRM197 de la toxine diphthérique et de la protéine de membrane externe de type 1 (OMP1) de *Neisseria meningitidis* ou de tout variant, analogue ou fragment de ces dernières ayant conservé la capacité de porteur. Les procédés permettant d'obtenir ces protéines sous forme purifiée sont bien connus de l'homme de l'art. Le terme "protéine" tel que utilisé dans la présente demande s'applique à toute chaîne d'acides aminés, quelque soit la longueur de la chaîne. En particulier, ce terme englobe la notion de peptide.

De manière générale, le groupe des protéines A1 à Am dans lequel on sélectionne de manière indépendante les protéines porteuses P1 à Pn, représente donc l'ensemble des protéines possédant un effet porteur. Pour ses besoins personnels, l'homme de l'art peut convenir que son choix se limitera à un nombre défini de protéines et en conséquence, il peut définir le groupe à partir duquel il effectuera sa sélection sur la base d'un nombre "m" de composants égal ou supérieur à 2 et au plus égal à "n" ; "n" étant tel que défini ci-avant. En particulier, l'homme de l'art peut déterminer le nombre minimal de protéines porteuses différentes qui est nécessaire, afin d'éviter le phénomène d'interférence. Pour ce faire, il prendra en compte la charge maximale à ne pas dépasser pour chacune des protéines porteuses. On appelle "charge maximale" la quantité de protéine porteuse au-delà de laquelle on observe une réponse immune diminuée à l'encontre d'un ou des polyosides par comparaison avec une composition monovalente correspondante (conjugué pris séparément).

En particulier en ce qui concerne l'anatoxine diphthérique et l'anatoxine tétanique, on estime que de manière avantageuse, la quantité de ces protéines présentes dans une dose d'une composition selon l'invention ne doit pas excéder 200 et 50 µg respectivement ; une telle dose étant prévue pour administration chez un mammifère, de préférence un humain. De préférence, la charge en Dt est inférieure ou égale à 150, 120 ou 100 µg ; de manière tout particulièrement préférée, à 80 ou 60 µg. De préférence, la charge en Tt est inférieure ou égale à 35 ou 25 µg ; de manière tout particulièrement préférée, à 20 ou 10 µg.

Ainsi, on peut convenir que pour une composition ne mettant en oeuvre que deux protéines porteuses différentes, la sélection de ces protéines s'effectuera à partir d'un groupe constitué des protéines A1 et A2. De préférence, A1 et A2 peuvent être l'anatoxine diphthérique (Dt) et l'anatoxine tétanique (Tt) respectivement ou vice et versa.

Selon un mode particulier, une composition mettant en oeuvre uniquement deux protéines porteuses différentes se caractérise par une répartition équilibrée du nombre de polyosides conjugués à la première protéine porteuse et du nombre de polyosides conjugués à la deuxième protéine porteuse. Par exemple, lorsque "n" est un nombre pair, "n"/2 protéines porteuses P1 à Pn sont A1 et "n"/2 protéines porteuses P1 à Pn sont A2 ou lorsque "n" est un nombre impair, ("n"+1)/2 protéines porteuses P1 à Pn sont A1 et ("n"-1)/2 protéines porteuses P1 à Pn sont A2.

Un polyoside utile aux fins de la présente invention peut notamment être un polyoside d'origine bactérienne ou fongique. Il peut notamment s'agir d'un polyoside de *Streptococcus e*.*g*., *Streptococcus pneumoniae, Staphylococcus, Klebsiella, Salmonella e.g., Salmonella typhi, Escherichia, Shigella, Neisseria e.g., Neisseria meningitidis, Haemophilus e.g., Haemophilus influenzae, Moraxella, Vibrio cholerae* ou *Mycobacterium tuberculosis*.

Au sein d'une composition selon l'invention, les polyosides peuvent provenir d'espèces différentes ou bien tous provenir de la même espèce *e*.*g*., de la même espèce bactérienne, éventuellement de sérogroupes / sérotypes différents. Afin d'illustrer cette dernière possibilité on cite une composition selon l'invention destinée à vacciner contre les infections à pneumocoque, qui contient au moins 8 valences, de préférence 10 ou 11 valences choisies parmi les sérotypes 1, 3, 4, 5, 6B, 7F, 9V, 14, 18C, 19F et 23F.

Ainsi, une composition constituant un vaccin pneumocoque comprend avantageusement 10 ou 11 valences *e*.*g*., représentées par 10 ou 11 conjugués dans lesquels les polyosides sont tous différents entre eux et dérivent (ont pour origine) des sérotypes / sérogroupes choisis parmi les sérotypes 1, 3, 4, 5, 6B, 7F, 9V, 14, 18C, 19F et 23F de *S. pneumoniae*. Il peut notamment s'agir d'une composition qui comprend 10 ou 11 conjugués sélectionnés parmi :
- le polyoside de sérotype 1 couplé à la Tt ou à la Dt ;
- le polyoside de sérotype 3 couplé à la Dt ;
- le polyoside de sérotype 4 couplé à la Tt ;
- le polyoside de sérotype 5 couplé à la Tt ou à la Dt ;
- le polyoside de sérotype 6B couplé à la Dt ;
- le polyoside de sérotype 7F couplé à la Tt ou à la Dt ;
- le polyoside de sérotype 9V couplé à la Tt ;
- le polyoside de sérotype 14 couplé à la Dt ;
- le polyoside de sérotype 18C couplé à la Dt ;
- le polyoside de sérotype 19F couplé à la Tt ; et
- le polyoside de sérotype 23F couplé à la Tt.

Sous un autre aspect, une composition constituant un vaccin pneumocoque peut comprendre 10 ou 11 valences représentées par 12 à 22, notamment 12 à 15 conjugués, dans lesquels les polyosides dérivent des sérotypes choisis parmi les sérotypes 1, 3, 4, 5, 6B, 7F, 9V, 14, 18C, 19F et 23F ; composition dans laquelle des conjugués de même valence diffèrent entre eux par la protéine porteuse. Il peut notamment s'agir d'une composition qui comprend :
- le polyoside de sérotype 1 couplé à la Tt ;
- le polyoside de sérotype 3 couplé à la Dt ;
- le polyoside de sérotype 4 couplé à la Tt ;
- le polyoside de sérotype 5 couplé à la Tt ;
- le polyoside de sérotype 6B couplé à la Dt ;
- le polyoside de sérotype 6B couplé à la Tt ;
- le polyoside de sérotype 7F couplé à la Tt ;
- le polyoside de sérotype 9V couplé à la Tt ;
- le polyoside de sérotype 9V couplé à la Dt ;
- le polyoside de sérotype 14 couplé à la Dt ;
- le polyoside de sérotype 18C couplé à la Dt ;
- le polyoside de sérotype 18C couplé à la Tt ;
- le polyoside de sérotype 19F couplé à la Tt ;
- le polyoside de sérotype 23F couplé à la Tt ; et
- le polyoside de sérotype 23F couplé à la Dt.

Un tel polyoside peut être avantageusement extrait du microorganisme selon des procédés conventionnels et purifié de même. Ce polyoside peut être utilisé sous forme brute après extraction / purification. Ou bien encore il peut être fragmenté afin d'obtenir un polyoside de poids moléculaire moyen inférieur à celui du polyoside extrait à l'origine. Un procédé de fragmentation particulièrement avantageux est décrit dans WO 93/7178, incorporé par référence.

Un conjugué dans lequel un polyoside est couplé par liaison covalente à une protéine porteuse peut être obtenu selon des procédés conventionnels bien connus de l'homme de l'art. Il peut être fait usage de linker ou de spacer afin d'assurer la conjugaison. Selon le mode de conjugaison mis en oeuvre, le conjugué qui en résulte peut être un conjugué dans lequel le polyoside est lié à la protéine par une seule fonction chimique (type soleil ou néoglycoconjugué), ou par plusieurs fonctions (type pelote). Il est à la portée de l'homme de l'art déterminer le mode de conjugaison le plus approprié en fonction de la nature du polyoside et plus particulièrement des groupements chimiques portés par le polyoside qui peuvent être mis en jeu au cours de la réaction de conjugaison.

Une composition selon l'invention peut être fabriquée de manière conventionnelle. En particulier, elle peut être formulée avec un diluent ou un support acceptable d'un point de vue pharmaceutique *e*.*g*., de l'eau ou une solution saline. En outre la composition peut contenir des ingrédients usuels tel qu'un tampon ; un agent conservateur ou stabilisant ; un adjuvant tel qu'un composé d'aluminium *e*.*g*., un hydroxide d'aluminium, un phosphate d'aluminium ou un hydroxy phosphate d'aluminium ; et le cas échéant un excipient de lyophilisation. En général, ces produits peuvent être sélectionnés en fonction du mode et de la voie d'administration et selon les pratiques pharmaceutiques standard. Des porteurs ou des diluants appropriés ainsi que ce qui est indispensable à l'élaboration d'une composition pharmaceutique sont décrits dans *Remington's Pharmaceutical Sciences,* un livre de référence standard dans ce domaine.

Une composition selon l'invention peut être administrée par n'importe quelle voie conventionnelle en usage dans le domaine des vaccins, en particulier par voie systémique *i*.*e*., parentérale *e*.*g*., par voie sous-cutanée, intramusculaire, intradermique ou intraveineuse ; ou par voie muqueuse *e*.*g*., par voie orale ou nasale.

L'administration peut avoir lieu en dose unique ou répétée une ou plusieurs fois *e*.*g*. 1, 2 ou 3 fois, après un certain délai d'intervalle. Le dosage approprié varie en fonction de divers paramètres, par exemple du nombre de valences contenues dans la composition, de la nature du ou des polyosides utilisés ou du mode d'administration. A titre indicatif on indique que de bons résultats peuvent être obtenus avec par valence, une dose de polyoside de 0.5 à 100 µg, de préférence de 1 à 50 µg, de manière tout à fait préférée de 1 à 10 µg. Une dose de la composition selon l'invention peut être avantageusement sous un volume de 0.1 à 2 ml.

Ci-après on présente à titre d'exemple, différents vaccins pneumocoque à valences multiples ; les valences étant choisies parmi les sérotypes 1, 3, 4, 5, 6B, 7F, 9V, 14, 18C, 19F et 23F. Les polyosides issus de ces sérotypes ont été fragmentés selon le procédé décrit dans WO 93/07178. Les polyosides couplés à la Tt (sauf le polyoside de type 1), l'ont été selon le procédé de conjugaison décrit dans WO 93/07178. Brièvement, un polyoside est soumis à une amination réductrice en présence de cyanoborohydrure de sodium afin de lier une molécule de diaminohexane à un groupe réducteur terminal. Puis le polyoside ainsi dérivé est activé par un groupe succinimide en utilisant du disuccinimidyl suberate (DSS). Le polyoside ainsi activé est mis à réagir directement avec la protéine porteuse. Le polyoside de sérotype 1 a été couplé à la Dt ou à la Tt selon le procédé de conjugaison décrit dans le brevet US n° 5,204,098. Les autres polyosides couplés à la Dt, l'ont été comme suit : Des groupes hydrazide sont incorporés sur le polyoside en faisant réagir le polyoside avec un excès d'acide adipique dihydrazide (ADH) en présence d'éthyl diméthyl amino propyl carbodiimide (EDAC) et de cyanoborohydrure de sodium (pour tous les types sauf le 3) ou simplement en présence de cyanoborohydrure de sodium (pour le type 3). Le polyoside ainsi dérivé est mis à réagir avec la protéine porteuse en présence d'EDAC. Les conditions expérimentales ont été contrôlées de manière à ce que l'on obtienne des conjugués dans lesquels la quantité de protéine soit comprise entre 1 et 4 fois, de préférence 2 fois, la valeur de la quantité de polyoside. Ainsi, pour une dose, 3 µg d'un polyoside particulier sont couplés à environ 6 µg de Dt et 1 µg d'un polyoside particulier est couplé à environ 2 µg de Tt.

La Dt et la Tt utilisées ont été préparées par détoxification au formaldéhyde à partir des toxines extraites respectivement de *Corynebacterium diphtheriae* et de *Clostridium tetani*.

Les formulations contiennent un tampon phosphate (0.475 mg d'ion PO₄²⁻ par dose) et du chlorure de sodium (4.5 mg par dose) et peuvent être adjuvantées par de l'hydroxyde d'aluminium (alum) (300 µg d'ion Al³⁺ par dose) et contenir un conservateur tel que le phénoxy éthanol formol. Une dose est sous un volume de 0.5 ml.

### EXEMPLE 1 : Formulation octavalente

| **Protéine porteuse** | **Polyoside** | |
|---|---|---|
| | Sérotype | Quantité pour une dose |
| Dt | 3 | 3 µg |
| Tt | 4 | 1 µg |
| Dt | 6B | 10 µg |
| Tt | 9V | 1 µg |
| Dt | 14 | 3 µg |
| Dt | 18C | 3 µg |
| Tt | 19F | 1 µg |
| Tt | 23F | 1 µg |

### EXEMPLE 2 : Formulations F3, F4 et F3bis à 11 valences

| **Protéine porteuse** | **Polyoside** | | | |
|---|---|---|---|---|
| | Sérogroupe / Sérotype | Quantité pour une dose de formulation F3 (µg) | Quantité pour une dose de formulation F4 (µg) | Quantité pour une dose de formulation F3 bis (µg) |
| Tt | 1 | 1 | - | 1 |
| Dt | 1 | - | 3 | - |
| Dt | 3 | 3 | 3 | 3 |
| Tt | 4 | 1 | 1 | 1 |
| Tt | 5 | 1 | - | 1 |
| Dt | 5 | - | 3 | |
| Dt | 6B | 10 | 10 | 3 |
| Tt | 6B | - | - | 1 |
| Tt | 7F | 1 | - | 1 |
| Dt | 7F | - | 3 | - |
| Tt | 9V | 1 | 1 | 1 |
| Dt | 9V | - | - | 3 |
| Dt | 14 | 3 | 3 | 3 |
| Dt | 18C | 3 | 3 | 3 |
| Tt | 18C | - | - | 1 |
| Tt | 19F | 1 | 1 | 1 |
| Tt | 23F | 1 | 1 | 1 |
| Dt | 23F | - | - | 3 |

La charge approximative en protéine dans chacune des trois formulations est comme suit :

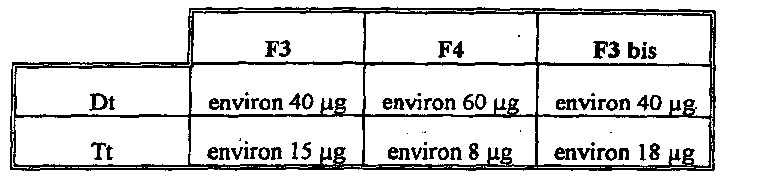

(ce qui correspond à un rapport pondéral protéine/polyoside de 2 environ).

## Revendications

1. Une composition comprenant "n" conjugués C1 à Cn ; chaque conjugué étant composé (i) d'un polyoside issu d'un sérotype / sérogroupe de *Streptococcus pneumoniae*, S1 à Sn respectivement et, (ii) d'une protéine porteuse P1 à Pn respectivement ; "n" étant un nombre égal ou supérieur à 2 ; composition dans laquelle les p olyosides S 1 à S n s ont identiques o u différents et da ns laquelle les protéines porteuses P1 à Pn sont sélectionnées de manière indépendante parmi un groupe constitué de "m" protéines porteuses A1 à Am ; "m" étant un nombre égal ou supérieur à 2 ; à condition qu'au moins une des protéines porteuses P1 à Pn soit différente des autres.

2. Une composition selon la revendication 1, dans laquelle les conjugués C1 à Cn sont tous différents entre eux soit par leur polyoside, soit par leur protéine porteuse ou par leur polyoside et leur protéine porteuse.

3. Une composition selon la revendication 2, dans laquelle les polyosides S1 à Sn sont tous différents entre eux.

4. Une composition selon la revendication 1, 2 ou 3, dans laquelle "n" est un nombre égal ou supérieur à 6.

5. Une composition selon la revendication 4, dans laquelle "n" est un nombre égal ou supérieur à 10.

6. Une composition selon l'une des revendications 1 à 5, dans laquelle les protéines porteuses P1 à Pn sont sélectionnées de manière indépendante parmi un groupe constitué de deux protéines porteuses A1 et A2.

7. Une composition selon la revendication 6, dans laquelle, lorsque "n" est un nombre pair, "n"/2 protéines porteuses P1 à Pn sont A1 et "n"/2 protéines porteuses P1 à Pn sont A2 ou lorsque "n" est un nombre impair, ("n"+1)/2 protéines porteuses P1 à Pn sont A1 et ("n"-1)/2 protéines porteuses P1 à Pn sont A2.

8. Une composition selon l'une des revendications 1 à 7, dans laquelle au moins une des protéines porteuses P1 à Pn est l'anatoxine diphthérique (Dt) et au moins une des protéines porteuses P1 à Pn est l'anatoxine tétanique (Tt).

9. Une composition selon la revendication 8, dans laquelle les protéines porteuses P1 à Pn sont sélectionnées parmi le groupe constitué par la Dt et la Tt.

10. Une composition selon la revendication 8 ou 9, dans laquelle la quantité de Dt est inférieure ou égale à 200 µg / dose.

11. Une composition selon la revendication 8, 9 ou 10, dans laquelle la quantité de Tt est inférieure ou égale à 50 µg / dose.

12. Une composition selon la revendication 1, qui comprend 10 ou 11 valences représentées par 10 ou 11 conjugués dans lesquels les polyosides sont tous différents entre eux et dérivent des sérotypes choisis parmi les sérotypes 1, 3, 4, 5, 6B, 7F, 9V, 14, 18C, 19F et 23F de *S. pneumoniae*.

13. Une composition selon la revendication 12, qui comprend 10 ou 11 conjugués sélectionnés parmi :
- le polyoside de sérotype 1 couplé à la Tt ou à la Dt ;
- le polyoside de sérotype 3 couplé à la Dt ;
- le polyoside de sérotype 4 couplé à la Tt ;
- le polyoside de sérotype 5 couplé à la Tt ou à la Dt ;
- le polyoside de sérotype 6B couplé à la Dt ;
- le polyoside de sérotype 7F couplé à la Tt ou à la Dt ;
- le polyoside de sérotype 9V couplé à la Tt ;
- le polyoside de sérotype 14 couplé à la Dt ;
- le polyoside de sérotype 18C couplé à la Dt ;
- le polyoside de sérotype 19F couplé à la Tt ; et
- le polyoside de sérotype 23F couplé à la Tt.

14. Une composition selon la revendication 1, qui comprend 10 ou 11 valences représentées par 12 à 22, notamment 12 à 15 conjugués, dans lesquels les polyosides dérivent des sérotypes choisis parmi les sérotypes 1, 3, 4, 5, 6B, 7F, 9V, 14, 18C, 19F et 23F ; composition dans laquelle des conjugués de même valence diffèrent entre eux par la protéine porteuse.

15. Une composition selon la revendication 14, qui comprend :
- le polyoside de sérotype 1 couplé à la Tt ;
- le polyoside de sérotype 3 couplé à la Dt ;
- le polyoside de sérotype 4 couplé à la Tt ;
- le polyoside de sérotype 5 couplé à la Tt ;
- le polyoside de sérotype 6B couplé à la Dt ;
- le polyoside de sérotype 6B couplé à la Tt ;
- le polyoside de sérotype 7F couplé à la Tt ;
- le polyoside de sérotype 9V couplé à la Tt ;
- le polyoside de sérotype 9V couplé à la Dt ;
- le polyoside de sérotype 14 couplé à la Dt ;
- le polyoside de sérotype 18C couplé à la Dt ;
- le polyoside de sérotype 18C couplé à la Tt ;
- le polyoside de sérotype 19F couplé à la Tt ;
- le polyoside de sérotype 23F couplé à la Tt ; et
- le polyoside de sérotype 23F couplé à la Dt.

16. Une composition qui comprend "n" conjugués C1 à Cn, chaque conjugué étant composé (i) d'un polyoside S1 à Sn respectivement et, (ii) d'une protéine porteuse P1 à Pn respectivement, "n" étant un nombre égal ou supérieur à 2 ; composition dans laquelle les polyosides S1 à Sn sont identiques ou différents et dans laquelle les protéines porteuses P1 à Pn sont sélectionnées de manière indépendante parmi un groupe constitué des anatoxines diphthérique (Dt) et tétanique (Tt), à condition qu'au moins une des protéines porteuses P1 à Pn soit différente des autres ; et qui est **caractérisée en ce que** la quantité en Dt et Tt est respectivement inférieure ou égale à 80 et 25 µg / dose.

17. Une composition selon la revendication 16, dans laquelle les conjugués C1 à Cn sont tous différents entre eux soit par leur polyoside, soit par leur protéine porteuse ou par leur polyoside et leur protéine porteuse.

18. Une composition selon la revendication 17, dans laquelle les polyosides S1 à Sn sont tous différents entre eux.

19. Une composition selon la revendication 16, 17 ou 18, dans laquelle "n" est un nombre égal ou supérieur à 6.

20. Une composition selon la revendication 19, dans laquelle "n" est un nombre égal ou supérieur à 10.

21. Une composition selon l'une des revendications 16 à 20, dans laquelle les polyosides S1 à Sn sont d'origine bactérienne.

22. Une composition selon la revendication 21, dans laquelle les polyosides S1 à Sn sont tous dérivés de la même espèce bactérienne.

23. Une composition selon la revendication 22, dans laquelle les polyosides S1 à Sn sont tous dérivés de l'espèce *Streptococcus pneumoniae*.

## Patentansprüche

1. Eine Zusammensetzung, umfassend "n" Konjugate C1 bis Cn; wobei jedes Konjugat besteht aus (i) jeweils einem Polysaccharid S1 bis Sn, das aus einem Serotyp / einer Serogruppe von *Streptococcus pneumoniae* hervorgegangen ist, und aus (ii) jeweils einem Trägerprotein P1 bis Pn; wobei "n" eine Zahl gleich oder größer 2 ist; eine Zusammensetzung, in der die Polysaccharide S1 bis Sn gleich oder verschieden sind und in der die Trägerproteine P1 bis Pn unabhängig ausgewählt sind aus einer Gruppe, die besteht aus "m" Trägerproteinen A1 bis Am; wobei "m" eine Zahl gleich oder größer 2 ist; unter der Bedingung, dass wenigstens eines der Trägerproteine P1 bis Pn sich von den anderen unterscheidet.

2. Eine Zusammensetzung gemäß Anspruch 1, in der die Konjugate C1 bis Cn sich alle entweder durch ihr Polysaccharid oder durch ihr Trägerprotein oder durch ihr Polysaccharid und ihr Trägerprotein voneinander unterscheiden.

3. Eine Zusammensetzung gemäß Anspruch 2, in der die Polysaccharide S1 bis Sn sich alle voneinander unterscheiden.

4. Eine Zusammensetzung gemäß Anspruch 1, 2 oder 3, in der "n" eine Zahl gleich oder größer 6 ist.

5. Eine Zusammensetzung gemäß Anspruch 4, in der "n" eine Zahl gleich oder größer 10 ist.

6. Eine Zusammensetzung gemäß einem der Ansprüche 1 bis 5, in der die Trägerproteine P1 bis Pn unabhängig ausgewählt sind aus einer Gruppe, die aus zwei Trägerproteinen A1 und A2 besteht.

7. Eine Zusammensetzung gemäß Anspruch 6, in der, wenn "n" eine gerade Zahl ist, "n"/2 Trägerproteine P1 bis Pn A1 sind und "n"/2 Trägerproteine P1 bis Pn A2 sind, oder wenn "n" eine ungerade Zahl ist, ("n" + 1)/2 Trägerproteine P1 bis Pn A1 sind und ("n" - 1)/2 Trägerproteine P1 bis Pn A2 sind.

8. Eine Zusammensetzung gemäß einem der Ansprüche 1 bis 7, in der wenigstens eins der Trägerproteine P1 bis Pn das Diphtherie-Anatoxin (Dt) ist und wenigstens eins der Trägerproteine P1 bis Pn das Tetanus-Anatoxin (Tt) ist.

9. Eine Zusammensetzung gemäß Anspruch 8, in der die Trägerproteine P1 bis Pn aus der Gruppe ausgewählt sind, die aus Dt und Tt besteht.

10. Eine Zusammensetzung gemäß Anspruch 8 oder 9, in der die Menge an Dt kleiner oder gleich 200 µg / Dosis ist.

11. Eine Zusammensetzung gemäß Anspruch 8, 9 oder 10, in der die Menge an Tt kleiner oder gleich 50 µg / Dosis ist.

12. Eine Zusammensetzung gemäß Anspruch 1, die 10 oder 11 Valenzen umfasst, repräsentiert von 10 oder 11 Konjugaten, in denen die Polysaccharide sich alle voneinander unterscheiden und zurückgehen auf die Serotypen, die ausgewählt sind unter den Serotypen 1, 3, 4, 5, 6B, 7F, 9V, 14, 18C, 19F und 23 F von *S*. *pneumoniae*.

13. Eine Zusammensetzung gemäß Anspruch 12, die 10 oder 11 Konjugate umfasst, die ausgewählt sind unter:
- dem Polysaccharid vom Serotyp 1, gekoppelt an Tt oder an Dt;
- dem Polysaccharid vom Serotyp 3, gekoppelt an Dt;
- dem Polysaccharid vom Serotyp 4, gekoppelt an Tt;
- dem Polysaccharid vom Serotyp 5, gekoppelt an Tt oder an Dt;
- dem Polysaccharid vom Serotyp 6B, gekoppelt an Dt;
- dem Polysaccharid vom Serotyp 7F, gekoppelt an Tt oder an Dt;
- dem Polysaccharid vom Serotyp 9V, gekoppelt an Tt;
- dem Polysaccharid vom Serotyp 14, gekoppelt an Dt;
- dem Polysaccharid vom Serotyp 18C, gekoppelt an Dt;
- dem Polysaccharid vom Serotyp 19F, gekoppelt an Tt; und
- dem Polysaccharid vom Serotyp 23F, gekoppelt an Tt.

14. Eine Zusammensetzung gemäß Anspruch 1, die 10 oder 11 Valenzen umfasst, repräsentiert von 12 bis 22, insbesondere 12 bis 15 Konjugaten, in denen die Polysaccharide auf die Serotypen zurückgehen, die ausgewählt sind unter den Serotypen 1, 3, 4, 5, 6B, 7F, 9V, 14, 18C, 19F und 23 F; eine Zusammensetzung, in der Konjugate der gleichen Valenz sich voneinander durch das Trägerprotein unterscheiden.

15. Eine Zusammensetzung gemäß Anspruch 14, die umfasst:
- das Polysaccharid vom Serotyp 1, gekoppelt an Tt;
- das Polysaccharid vom Serotyp 3, gekoppelt an Dt;
- das Polysaccharid vom Serotyp 4, gekoppelt an Tt;
- das Polysaccharid vom Serotyp 5, gekoppelt an Tt;
- das Polysaccharid vom Serotyp 6B, gekoppelt an Dt;
- das Polysaccharid vom Serotyp 6B, gekoppelt an Tt;
- das Polysaccharid vom Serotyp 7F, gekoppelt an Tt;
- das Polysaccharid vom Serotyp 9V, gekoppelt an Tt;
- das Polysaccharid vom Serotyp 9V, gekoppelt an Dt;
- das Polysaccharid vom Serotyp 14, gekoppelt an Dt;
- das Polysaccharid vom Serotyp 18C, gekoppelt an Dt;
- das Polysaccharid vom Serotyp 18C, gekoppelt an Tt;
- das Polysaccharid vom Serotyp 19F, gekoppelt an Tt;
- das Polysaccharid vom Serotyp 23F, gekoppelt an Tt; und
- das Polysaccharid vom Serotyp 23 F, gekoppelt an Dt.

16. Eine Zusammensetzung, die "n" Konjugate C1 bis Cn umfasst; wobei jedes Konjugat besteht aus (i) jeweils einem Polysaccharid S1 bis Sn und aus (ii) jeweils einem Trägerprotein P1 bis Pn; wobei "n" eine Zahl gleich oder größer 2 ist, eine Zusammensetzung, in der die Polysaccharide S1 bis Sn gleich oder verschieden sind und in der die Trägerproteine P1 bis Pn unabhängig ausgewählt sind aus einer Gruppe, die aus dem Diphtherie-Anatoxin (Dt) und dem Tetanus-Anatoxin (Tt) besteht; unter der Bedingung, dass wenigstens eines der Trägerproteine P1 bis Pn sich von den anderen unterscheidet, und die **dadurch gekennzeichnet ist, dass** die Menge an Dt und Tt kleiner oder gleich 80 beziehungsweise 25 µg / Dosis ist.

17. Eine Zusammensetzung gemäß Anspruch 16, in der die Konjugate C1 bis Cn sich alle entweder durch ihr Polysaccharid oder durch ihr Trägerprotein oder durch ihr Polysaccharid und ihr Trägerprotein voneinander unterscheiden.

18. Eine Zusammensetzung gemäß Anspruch 17, in der die Polysaccharide S1 bis Sn sich alle voneinander unterscheiden.

19. Eine Zusammensetzung gemäß Anspruch 16, 17 oder 18, in der "n" eine Zahl gleich oder größer 6 ist.

20. Eine Zusammensetzung gemäß Anspruch 19, in der "n" eine Zahl gleich oder größer 10 ist.

21. Eine Zusammensetzung gemäß einem der Ansprüche 16 bis 20, in der die Polysaccharide S1 bis Sn bakteriellen Ursprungs sind.

22. Eine Zusammensetzung gemäß Anspruch 21, in der die Polysaccharide S1 bis Sn alle auf die gleiche Bakterienspezies zurückgehen.

23. Eine Zusammensetzung gemäß Anspruch 22, in der die Polysaccharide S1 bis Sn alle auf die Spezies *Streptococcus pneumoniae* zurückgehen.

## Claims

1. Composition comprising "n" conjugates C1 to Cn, each conjugate being composed (i) of a polysaccharide derived from a *Streptococcus pneumoniae* serotype/serogroup S1 to Sn respectively, and (ii) of a carrier protein P1 to Pn respectively; "n" being a number equal to or greater than 2; in which composition the polysaccharides S1 to Sn are identical or different and in which the carrier proteins P1 to Pn are selected independently from a group consisting of "m" carrier proteins A1 to Am, "m" being a number equal to or greater than 2, provided that at least one of the carrier proteins P1 to Pn is different from the others.

2. Composition according to Claim 1, in which the conjugates C1 to Cn are all different from each other either by their polysaccharide or by their carrier protein or by their polysaccharide and their carrier protein.

3. Composition according to Claim 2, in which the polysaccharides S1 to Sn are all different from each other.

4. Composition according to Claim 1, 2 or 3, in which "n" is a number equal to or greater than 6.

5. Composition according to Claim 4, in which "n" is a number equal to or greater than 10.

6. Composition according to one of Claims 1 to 5, in which the carrier proteins P1 to Pn are independently selected from a group consisting of two carrier proteins A1 and A2.

7. Composition according to Claim 6, in which when "n" is an even number, "n"/2 carrier proteins P1 to Pn are A1 and "n"/2 carrier proteins P1 to Pn are A2 or when "n" is an odd number, ("n"+1)/2 carrier proteins P1 to Pn are A1 and ("n"-1)/2 carrier proteins P1 to Pn are A2.

8. Composition according to one of Claims 1 to 7, in which at least one of the carrier proteins P1 to Pn is the diphtheria toxoid (Dt) and at least one of the carrier proteins P1 to Pn is the tetanus toxoid (Tt).

9. Composition according to Claim 8, in which the carrier proteins P1 to Pn are selected from the group consisting of Dt and Tt.

10. Composition according to Claim 8 or 9, in which the quantity of Dt is less than or equal to 200 µg/dose.

11. Composition according to Claim 8, 9 or 10, in which the quantity of Tt is less than or equal to 50 µg/dose.

12. Composition according to Claim 1, which comprises 10 or 11 valencies represented by 10 or 11 conjugates in which the polysaccharides are all different from each other and are derived from the serotypes chosen from serotypes 1, 3, 4, 5, 6B, 7F, 9V, 14, 18C, 19F and 23F of *S*. *pneumoniae*.

13. Composition according to Claim 12, which comprises 10 or 11 conjugates selected from:
- serotype 1 polysaccharide coupled to Tt or to Dt;
- serotype 3 polysaccharide coupled to Dt;
- serotype 4 polysaccharide coupled to Tt;
- serotype 5 polysaccharide coupled to Tt or to Dt;
- serotype 6B polysaccharide coupled to Dt;
- serotype 7F polysaccharide coupled to Tt or to Dt;
- serotype 9V polysaccharide coupled to Tt;
- serotype 14 polysaccharide coupled to Dt;
- serotype 18C polysaccharide coupled to Dt;
- serotype 19F polysaccharide coupled to Tt; and
- serotype 23F polysaccharide coupled to Tt.

14. Composition according to Claim 1, which comprises 10 or 11 valencies represented by 12 to 22, especially 12 to 15 conjugates, in which the polysaccharides are derived from the serotypes chosen from serotypes 1, 3, 4, 5, 6B, 7F, 9V, 14, 18C, 19F and 23F; in which composition conjugates of the same valency differ from each other in the carrier protein.

15. Composition according to Claim 14, which comprises:
- serotype 1 polysaccharide coupled to Tt;
- serotype 3 polysaccharide coupled to Dt;
- serotype 4 polysaccharide coupled to Tt;
- serotype 5 polysaccharide coupled to Tt;
- serotype 6B polysaccharide coupled to Dt;
- serotype 6B polysaccharide coupled to Tt;
- serotype 7F polysaccharide coupled to Tt;
- serotype 9V polysaccharide coupled to Tt;
- serotype 9V polysaccharide coupled to Dt;
- serotype 14 polysaccharide coupled to Dt;
- serotype 18C polysaccharide coupled to Dt;
- serotype 18C polysaccharide coupled to Tt;
- serotype 19F polysaccharide coupled to Tt;
- serotype 23F polysaccharide coupled to Tt; and
- serotype 23F polysaccharide coupled to Dt.

16. Composition which comprises "n" conjugates C1 to Cn, each conjugate being composed (i) of a polysaccharide S1 to Sn respectively and (ii) of a carrier protein P1 to Pn respectively, "n" being a number equal to or greater than 2; in which composition the polysaccharides S1 to Sn are identical or different and in which the carrier proteins P1 to Pn are selected independently from a group consisting of diphtheria (Dt) and tetanus (Tt) toxoids, provided that at least one of the carrier proteins P1 to Pn is different from the others; and which is **characterized in that** the quantity of Dt and Tt is respectively less than or equal to 80 and 25 µg/dose.

17. Composition according to Claim 16, in which the conjugates C1 to Cn are all different from each other either by their polysaccharide or by their carrier protein or by their polysaccharide and their carrier protein.

18. Composition according to Claim 17, in which the polysaccharides S1 to Sn are all different from each other.

19. Composition according to Claim 16, 17 or 18, in which "n" is a number equal to or greater than 6.

20. Composition according to Claim 19, in which "n" is a number equal to or greater than 10.

21. Composition according to one of Claims 16 to 20, in which the polysaccharides S1 to Sn are of bacterial origin.

22. Composition according to Claim 21, in which the polysaccharides S1 to Sn are all derived from the same bacterial species.

23. Composition according to Claim 22, in which the polysaccharides S1 to Sn are all derived from the species *Streptococcus pneumoniae*.
